Europäisches Patentamt

European Patent Office

Office européen des brevets

(11) Publication number: **0 289 361 B1**

# EUROPEAN PATENT SPECIFICATION

(45) Date of publication of patent specification: **02.12.92**  (51) Int. Cl.[5]: **A61M 5/14**

(21) Application number: **88303965.3**

(22) Date of filing: **29.04.88**

(54) **Medical infusion apparatus.**

(30) Priority: **01.05.87 GB 8710441**

(43) Date of publication of application:
**02.11.88 Bulletin 88/44**

(45) Publication of the grant of the patent:
**02.12.92 Bulletin 92/49**

(84) Designated Contracting States:
**AT BE CH DE ES FR GB GR IT LI LU NL SE**

(56) References cited:
EP-A- 0 012 389      WO-A-81/01658
WO-A-86/03415        FR-A- 2 441 150
US-A- 3 731 679      US-A- 3 807 228
US-A- 4 321 461      US-A- 4 372 304
US-A- 4 384 578      US-A- 4 613 327

(73) Proprietor: **Product Innovation Holdings Limited
Mill Studios Crane Mead
Ware, Hertfordshire SG12 9PY(GB)**

(72) Inventor: **Frank, Peter
39 St. Gabriel's Road
London N.W.2(GB)**
Inventor: **Giles, Terence George
54 The Mount
Coulsdon, Surrey CR3 2PY(GB)**

(74) Representative: **Burke, Steven David et al
R.G.C. Jenkins & Co. 26 Caxton Street
London SW1H 0RJ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to medical infusion apparatus.

In general, there are currently two types of medical infusion apparatus. The first type is used for injecting very small doses of drugs into a patient over long periods. Such devices require very accurate control of a mechanism used to pressurise the liquid (e.g. a mechanism used to operate a syringe plunger), and the devices have to be arranged so that even very small variations from the set flow rate, which can be very dangerous, cannot occur. The devices are consequently designed only for very small flow rates, and are very expensive.

The present invention is concerned primarily with the other type of infusion apparatus, which is used for injecting relatively large quantities of liquid, e.g. saline, blood, etc. Although it is necessary for the flow to be carefully controlled, the very high degree of accuracy required for the first type of apparatus is not needed. The most common apparatus of the second type is the gravity-operated drip-feed. This apparatus comprises a stand from which is suspended a liquid reservoir, e.g. in the form of a bag of plastics material. Liquid is fed under gravity from the reservoir to the patient via a tube which is formed with a drip chamber. A clamp is provided downstream of the drip chamber, and can be adjusted so as to vary the flow restriction caused by the clamp, and thus vary the flow rate. The flow rate is estimated by counting the rate of drips in the drip chamber. This is an inexpensive arrangement, which is important especially for hospitals with a limited budget. However, there are a number of disadvantages. Setting up the apparatus is time consuming, and requires priming of the drip chamber which,if not done carefully, could result in problems such as air bubbles in the liquid being delivered to the patient. The apparatus is very clumsy, and makes movement of the patient awkward. The control of flow rate is very inaccurate, and this problem is exacerbated by the fact that it has to be checked regularly by a nurse. If the nurse is busy there can be fairly long periods between the checking of the apparatus. In the meantime, the cannula used to inject fluid into the patient could have shifted so that fluid is incorrectly being injected into tissue rather than, e.g. a vein. When the nurse does manage to check the apparatus, it takes several seconds at least to ensure that the flow rate is correct, and even longer if it is found that it needs adjustment.

In addition, although the cost of the apparatus as a whole is relatively low, the giving set, i.e. the tube leading from the reservoir to the patient and including the drip chamber and possibly a filter, is usually replaced after each usage, and therefore the cost of using the apparatus tends to be very high.

In some circumstances the apparatus is not sufficiently accurate. In these cases, the tendency is to use a peristaltic pump for liquid infusion. This gives highly accurate results, but is very expensive, and therefore few such devices are normally present in a hospital. The apparatus also requires significant amounts of power which makes transport difficult and, if battery-powered, requires that the apparatus be checked regularly to find out whether the batteries need replacement. The apparatus is arranged to deliver liquid using either a standard drip-feed giving set, thus encountering some of the problems mentioned above, or silicon tubing, which can be made to accurate dimensions but which is expensive.

Another known form of apparatus uses a sensor for counting drips and a regulator to control flow in response to the drip rate. This also has the problem of the drip chamber making the cost of usage high, and has a fairly high current consumption.

There have been various proposals for dealing with these problems, but they suffer to some extent from the disadvantages referred to, and have not met with commercial success.

US Patent 4613327 discloses a medical infusion apparatus which is said to be compact and portable and to produce little noise. It would, however, be desirable to provide apparatus in which these factors are considerably improved, in which the power requirements are substantially decreased, and in which it is ensured that liquid is administered to patients in the correct amounts.

It is desirable in particular for the flow monitor of the apparatus to be inexpensive and accurate, and to have a low power consumption. Known flow monitors for infusion apparatus include drop sensors, such as that shown in WO 86/03415. These need to operate continuously and therefore consume large amounts of current. They also increase the cost of the disposable parts of the apparatus, because of the provision of a drip chamber, resulting in very high usage costs. Another flow sensor is disclosed in US Patent 4384578. The sensor disclosed here employs tubing which has spaced-apart tubular metal segments, the temperatures of which are sensed by thermistors. A resistor is provided to apply heat to the downstream segment. The current required to maintain a constant temperature differential is measured, and used as an indication of fluid flow. Such an arrangement also requires large amounts of current, is of low accuracy, and is affected by ambient temperatures so that thermal insulation of parts of the apparatus is required. In addition, the disposable part of the apparatus needs to include two separate metal segments forming part of the sensor.

In accordance with the present invention there is provided medical infusion apparatus comprising:

a liquid reservoir;

a conduit for delivering liquid from the reservoir to a patient; and

a monitor for monitoring the flow rate of said liquid along said conduit;

characterised in that said monitor comprises means external of said conduit for applying a pulse of heat to liquid at a location in the conduit and radiation sensing means disposed externally of the conduit for sensing a change in temperature of liquid at a position downstream of the location, so that the speed of travel of the heat pulse can be determined, whereby the monitor is non-intrusive to permit removal of the conduit from the monitor.

In effect, the speed at which the heat pulse travels down the conduit is detected in order to determine the flow rate. This arrangement can be embodied in various forms. For example, the time between the injection of the heat pulse and the sensing of the heat pulse having reached a predetermined position downstream can be monitored. Alternatively, the speed at which the heat pulse passes the sensing point can be monitored. In the preferred embodiment, however, instead of just one sensor there are two sensors downstream of the location at which the heat pulse is injected, and the time taken for the heat pulse to travel between the two sensors is monitored to give an indication of flow rate. This results in the measurement being independent of the time required for the initial heating of the liquid, which depends on factors which may vary.

The advantages of this aspect of the invention include the fact that the sensor can be made non-intrusive, so that the conduit is readily removable from the monitor and so as to reduce the possibility of contamination of the liquid. It may also be designed so that it requires very small amounts of current in operation, and needs to be operated only at relatively long intervals.

The heat pulse can be injected using a flexible resistive material disposed around the conduit and intermittently powered to generate heat. The material is preferably resilient, and may serve as a clip for retaining the conduit in position, and thus ensuring its close proximity to the heat source. The sensors are preferably responsive to infra-red radiation emitted by the liquid in the conduit.

In accordance with a preferred aspect of the invention there is provided a medical infusion apparatus comprising a housing for supporting the liquid reservoir and the conduit, the housing also supporting means for applying pressure to the reservoir to cause liquid to flow along the conduit, the flow monitor, and means responsive to the sensing means for regulating the flow. The housing preferably forms an enclosure within which the reservoir, the conduit, the pressurising means, the sensor, the regulator and a control circuit for the sensor and regulator are located. The enclosure preferably has an access member, such as a door or lid, which can be opened to permit replacement of the reservoir and/or conduit. As will be explained in more detail below, such an arrangement facilitates the provision of a self-contained and compact infusion apparatus which provides a properly controlled liquid flow.

An infusion apparatus according to the present invention is preferably electrically operated. However, it is very important that the apparatus consume very small amounts of current so that the apparatus can be made battery-operated and thus more portable, and preferably designed such that battery replacement is required only after relatively long periods of e.g. several months. It is desired that the current consumption be of the order of 3mA at 6 volts, so that the apparatus can be powered for several months at least by small dry-cell batteries. Several preferred aspects of the invention explained below are aimed at reducing the power consumption to such an order of magnitude, which has not previously been achieved in the type of medical infusion apparatus with which the present invention is concerned.

In accordance with one of these preferred aspects, the apparatus comprises means for intermittently monitoring the flow rate, and a control circuit responsive to the monitoring means for regulating the rate.

Preferably, the resilient pressurising means comprises a spring acting upon the flexible reservoir. The spring preferably provides a substantially constant force, and may be a sheet of, e.g., steel which can be unwound from a rolled form (or rolled up) so that as it rewinds (or unrolls) it gradually squeezes liquid from the reservoir. In the preferred embodiment, the apparatus has an enclosure such as that referred to above, and the opening of the access member of the enclosure withdraws the resilient pressurising means from the reservoir so as to facilitate the removal and replacement of the reservoir. Also, the opening of the access member preferably causes energy to be stored by the pressurising means. Preferably the closing of the access member causes the pressurising means to act against the reservoir.

In such an arrangement, power consumption is reduced by virtue of the type of pressurising means and by the intermittent monitoring of the flow rate. Preferably, the position of the regulator which determines the rate of flow of liquid from the apparatus is controlled by a motor, and is stable while the motor is de-energised. This further reduces power consumption by permitting a desired regulator position to be achieved by electrical control of the motor, while avoiding the need to maintain a supply of current to the regulating means in order to keep the regulator at the desired position. The motor may be an ordinary motor preferably coupled to the regulator by gears which produce a high gear ratio so that the pressure in the tube is insufficient to shift the regulator when the motor is de-energised. Alternatively, however, the motor may be a stepper motor.

Whenever the flow rate monitor determines that an alteration of the flow rate is required, the system is preferably arranged to drive the regulator using a signal which is determined by the amount by which the detected flow rate differs from the desired rate. This signal could for example control the amount of time for which the motor is operated, or the number of steps moved by the motor if this is a stepper motor. This is preferable to carrying out repeated incremental changes of regulator position, and each time checking the resulting altered flow rate, because the latter technique would require greater current consumption. However, if the interval between monitoring operations is relatively long in order to save power, it is important that the flow rate is corrected in a small number of stages. Accordingly, it is necessary for the response of the regulator to be reasonably predictable and repeatable.

It is also important that the regulator be designed so that the cost of use of the apparatus is not significantly increased. For this reason, it is preferred that the regulator be arranged to act upon a simple tube, such as the inexpensive P.V.C. tubes used in conventional giving sets. The regulator preferably squeezes the sides of the tube together and thereby controls the rate of flow.

Regulators of this type are disclosed in US Patents 3543752 and 4372304. In the former patent, plastic tubing is positioned between a regulator member and a rigid stationary base, and the regulator member is spring biased against the plastic tubing in order to close it. The regulator member can be shifted against the spring bias in order to open the tubing. The regulator is merely required to open and close the tubing. In US Patent 4372304, a stepper motor operates on a plunger via reduction gearing in order to cause axial movement of the plunger to squeeze a tube. In both these cases, it would be extremely difficult to achieve controllable flow rates with a predictable and repeatable response as is required for relatively infrequent adjustment of the regulator.

In the preferred embodiment of the invention, the regulator has a position which can be adjusted in order to regulate the flow of liquid from the apparatus, and comprises first and second members between which can be located the conduit carrying the liquid, the first and second members being movable toward each other in order to restrict liquid flow, and at least one of the members being resiliently biassed toward the other member. It has been found that by resiliently biassing one of the members, rather than having it fixed in position, the range of operation of the regulator which corresponds to the range of flow rates over which control is to be exercised is substantially increased, and the predictability and repeatability of the regulator response is substantially increased, without requiring special tubing. The consequence of this is that accurate alteration of the flow rate is more easily achieved, and by proper arrangement of the device a more linear control can be obtained. The conduit may be a simple tube, e.g. made of P.V.C.

Preferably, the arrangement is such that the tube is caused to adopt a substantially "U"-shaped configuration, with the first and second members engaging opposite sides of the tube at the base of the "U", and further members engaging the tube at respective sides of the base of the "U". Such an arrangement facilitates the complete shutting-off of the liquid flow, which is desired in certain circumstances.

A regulator of the above type has the further advantage that, despite manufacturing irregularities in both the infusion apparatus and the conduit, the predictability of the regulator operation is such that it is possible to ensure that a particular setting of the regulator upon start-up will result in an intermediate flow rate which permits monitoring but is not excessive for the patient.

In accordance with a still further preferred aspect of the invention, at least part of the conduit is an integrally formed member and is carried by but detachable from a housing of the apparatus, the conduit part and a support arrangement of the housing being configured such that in use they interengage to locate the conduit part at a predetermined location with respect to the fluid flow monitor of the apparatus. Preferably the conduit part is transparent to infra-red radiation and is located in proximity to at least one infrared sensor of the monitor. Preferably the support arrangement includes a heat-generating member serving as both part of the flow monitor and a locating means for the conduit part. Preferably the conduit part has a portion adapted for insertion into the reservoir. Such an arrangement permits the use of inexpensive conduits which do not have any built-in means for indicating flow rate but nevertheless ensure that the apparatus as a whole can monitor the flow rate accurately. Making the conduit inexpensive is very important as it is a part of the apparatus that is discarded after use. US Patent 4384578 also discloses a conduit having a part locatable at a predetermined position in the housing of a flow sensor. However, the part needs to include two metal segments with an intermediate piece of plastics tubing, and is remote from the reservoir.

Another preferred aspect of the invention is directed to the further reduction of the current required by a flow monitor. In accordance with this aspect, the flow rate monitor is arranged to operate periodically, so that the flow rate is monitored at predetermined intervals, the monitor having at least an active mode and a standby mode, the intervals between monitoring being shorter in the active mode than in the standby mode. The periodic operation of the sensor reduces the total current consumption. By having two modes of operation, it is possible to ensure that the flow rate is closely monitored at critical times, such as immediately after the apparatus is put into operation, while enabling greater reduction in current consumption during non-critical periods.

Preferably the monitor is actuated using a microprocessor forming part of a control circuit of the apparatus. The microprocessor preferably itself has active and standby modes in which it consumes respectively greater and smaller amounts of current. A timer may be provided for generating, at predetermined intervals, signals for causing the microprocessor to enter its active mode.

Preferably, the apparatus is arranged to detect one or both of the following conditions, in response to which the microprocessor is caused to enter its active mode: (a) a signal from a timer, as indicated above, to enable monitoring (and if necessary regulation) of the flow rate, and (b) a signal from a sensor detecting excessive amounts of air passing along the conduit so that the microprocessor can, for example, generate an alarm.

An apparatus of the present invention can be made less expensive to use than conventional equipment, and can provide better control. The extremely low current consumption also results in extremely portable equipment which requires little attention.

An arrangement according to the invention will now be described by way of example with reference to the accompanying drawings, in which:

Figure 1 is a perspective view, partly cut away, schematically showing a medical infusion apparatus according to the invention;

Figure 2 is a plan view of the apparatus of Figure 1;

Figure 3 is a schematic diagram of the circuit of the apparatus;

Figure 4 schematically illustrates a flow rate regulator of the apparatus; and

Figure 5 schematically illustrates a bubble detector of the apparatus.

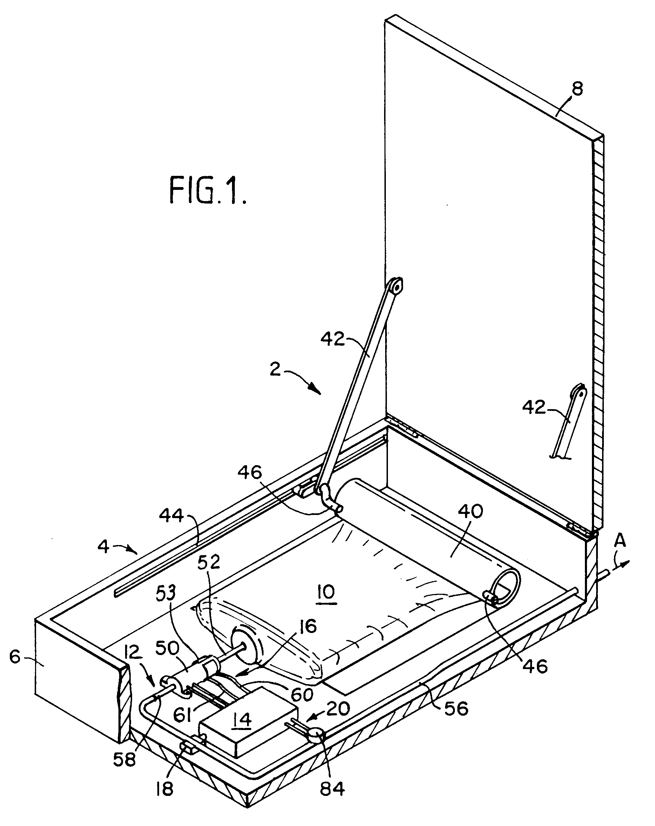

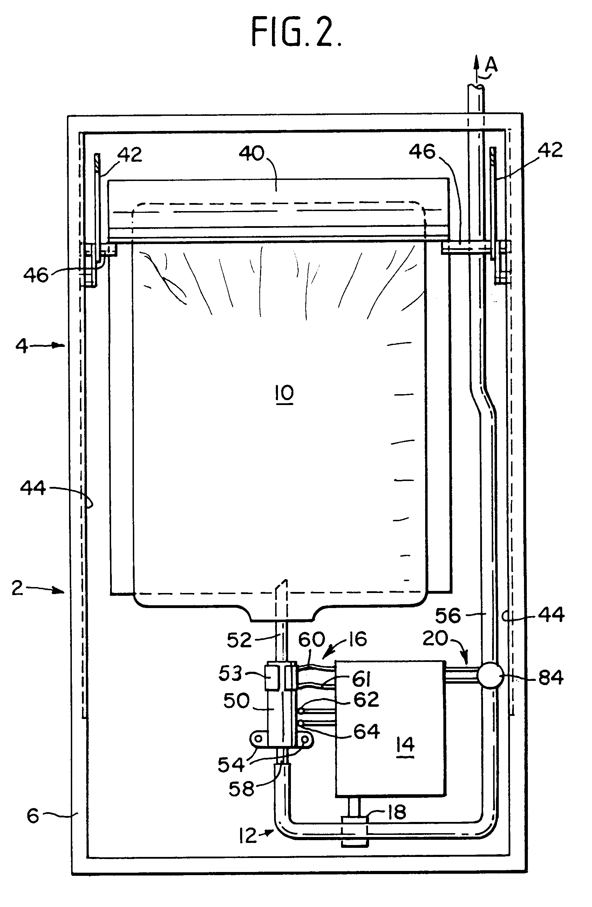

Referring to Figures 1 and 2, the apparatus 2 comprises a housing 4 having a base 6 and a hinged lid 8. The housing base 6 supports a reservoir 10 in the form of a flexible plastics bag of a standard type used in drip feed apparatus, and a conduit indicated generally at 12 which conveys liquid from the reservoir 10 in the direction of arrow A to a patient.

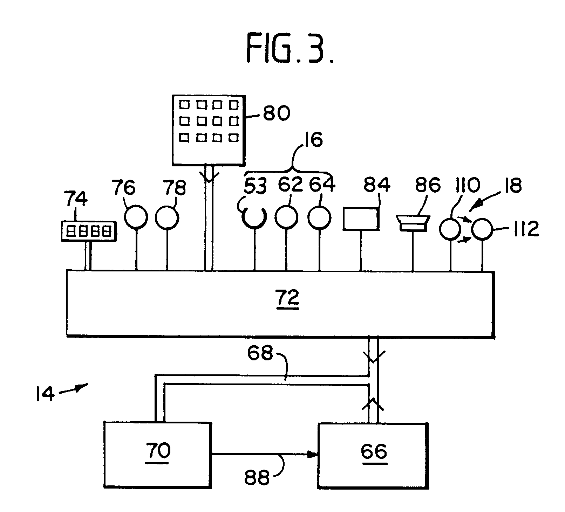

The housing base 6 also supports a control unit 14 coupled to a sensor arrangement 16, a bubble detector 18 and a flow rate regulator 20. In addition, the housing 4 has an externally-visible digital display, externally-visible warning lights, externally-operable controls, preferably in the form of a keyboard, and a sound transducer for emitting alarm sounds. These devices are not shown in Figures 1 and 2, but are indicated schematically in Figure 3 and are of types known per se for microprocessor-controlled appliances.

The base 6 also supports a spring 40 used to pressurise the reservoir 10. The spring comprises a steel sheet in a rolled configuration, and is shown in Figures 1 and 2 in a state in which it is substantially unrolled so that the reservoir 10 can be removed. In use, the spring 40 tends to roll up and thereby squeeze fluid from the reservoir 10.

The lid 8 has a pair of arms 42 hinged thereto at their upper ends. The lower ends are guided for movement substantially in the direction in which the spring 40 rolls and unrolls by slots 44. Extensions 46 fixed to the lower ends of the arms 42 are arranged to engage the spring 40 so that as the lid 8 is opened the spring 40 is caused to unroll. This supplies energy to the spring 40, and also moves it to the position in which the reservoir 10 can be removed. When the lid 8 is closed, the spring 40 is able to start rolling up and thus pressurising the reservoir 10. If desired, a latch could be provided to hold the lid 8 in its open position.

The spring 40 is of a type which tends to exert a constant force as it rolls up.

The conduit 12 includes a part 50 which is formed of a unitary molding. The part 50 is generally tubular and has an inlet end 52 forming a spike which is pointed so that it can be inserted in the usual way into the standard reservoir 10. The part 50 is fitted into a resilient "C"-shaped clip 53 fixed to the base 6 of the housing 4, which holds the part 50 in position. If desired, the central section of the part 50 may have flanges indicated at 54 with apertures for locating on pegs fixed to the base 6 of the housing 4. In any event, the part 50 is positioned in a predetermined location with respect to the sensor arrangement 16. The remainder of the conduit 12 comprises a simple tube 56 made of P.V.C. which is pushed onto the outlet end 58 of the part 50 and which extends past the bubble detector 18, the regulator 20 and then out of the housing 4.

Because the conduit 12 comprises a simple tube 56 and a molded part 50 (together with an in-line filter e.g. between the part 50 and the tube 56, if desired), the conduit is substantially less expensive than the conventional "giving sets" used in standard gravity-operated drip-feed apparatus, as a result of the fact that the disposable conduit need not include a drip-feed chamber, or an individual regulator. The part 50 is used both for insertion into the reservoir and, as explained below, enabling sensing of flow rate. It will be appreciated that even a small saving on the disposable parts of the apparatus quickly justifies the capital expense of the apparatus as a whole and thereafter results in very substantial cost savings.

The sensor arrangement 16 comprises a heat source formed by the clip 53 which is made of electrically resistive material, preferably hard wearing, such as cermet. This is connected to the control unit 14 by leads 60,61 so that electrical power can be applied to heat up the clip 53 and hence the liquid in the conduit part 50. The sensor arrangement 16 also comprises two pyro-electric sensors 62 and 64 (Mullard type No. RPY 100). Other sensors could be used, but passive infra-red sensors are preferred. The source 53 is, in use, energised for a brief period in order to heat up, by a very small amount, the liquid flowing through the conduit 12. This is then detected by the sensors 62 and 64. For this purpose, the material of the part 50 of the conduit 12 is selected so that it is substantially transparent to infra-red radiation. The material may for example be polyethylene. After the source 53 has been briefly energised, the output signals from the sensors 62 and 64 are checked. This can be done in a variety of ways. For example, the time between the occurrence of the peaks in the signals from the sensors can be measured, or alternatively the point at which the value of each output signal increases to a predetermined proportion (e.g. 80%) of its peak value can be measured, and the time between these points determined. The latter can be achieved by repeatedly sampling the outputs of the sensors 62 and 64, storing the samples, determining therefrom when the peak has been reached, and then determining the time at which the samples representing the predetermined proportion of the peak were taken. In any event, a value indicative of the rate at which fluid flows along the conduit is derived. This can if desired be converted into a measurement of flow, by for example using the derived value to select a location in a table stored in a memory of the control circuit, which table contains corresponding flow rate values. If the flow rate deviates from a selected rate, the control unit 14 operates regulator 20 in order to increase or decrease the flow rate, as appropriate.

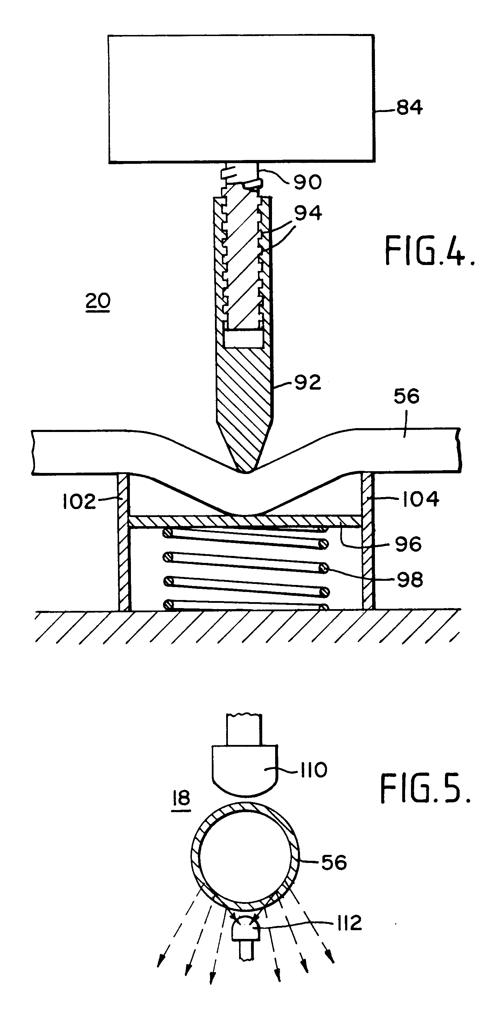

The regulator 20 is shown schematically in Figure 4. The regulator comprises a stepper motor 84 having an output shaft 90 which co-operates with a regulating member 92 by means of a helical thread 94, so that rotary movement of the shaft 90 is converted into linear movement of the member 92, in an upward/downward direction as shown in Figure 4.

The tube 56 passes between the regulating member 92 and an opposing member 96 which is biassed by a spring 98 toward the regulating member 92. The opposing member 96 engages the tube 56 at a point opposite that at which it is engaged by the regulating member 92. The total compression is thus determined by the position of regulating member 92 and the force of spring 98. This gives rise to the extended operating range of the regulator and the advantages mentioned above. Instead of or in addition to the spring 98, the member 92 could be replaced by a first member driven by the motor, and a second member pressed against the tube 56 by the first member via a spring.

Further members 102 and 104, which are fixedly positioned, engage the tube 56 at opposite sides of the point at which it is squeezed, so that the tube 56 adopts a substantially "U"-shaped configuration. Operation of the stepper motor 84 causes the regulating member 92 to move toward or away from the opposing member 96, thus varying the amount by which the tube 56 is pinched, and therefore varying the restriction to liquid flow. By having the tube in a "U"-shaped configuration, which gradually deepens as the flow is restricted, the tube can be more readily pinched to a sufficient extent as to shut-off the liquid flow.

The bubble detector 18 is shown in Figure 5. This comprises a radiation source 110, which could be a lamp, an LED, a source of infra-red radiation, etc. The source 110 is positioned on the opposite side of the tube 56 from a radiation sensor 112, which is arranged to detect radiation only in the central region of the tube.

The tube, which is made of transparent plastics material such as P.V.C., has parallel inner and outer walls. In the absence of liquid in the tube 56, radiation from the source 110 passes substantially straight through the tube 56, and leaves the tube in a wide variety of directions as indicated by the arrows shown in phantom. The radiation is generally diffuse, and little reaches the sensor 112.

However, when liquid is in the tube 56, it acts as a lens and focusses the radiation onto the sensor 112 as shown by the solid-line arrows. This results in an increase in the detected intensity. Air bubbles can thus be detected by looking for decreases in sensed radiation intensity.

Depending upon the wavelength of the radiation, there may be some liquids, e.g. blood, which have an optical density such that air bubbles result in an increase in the sensed radiation. In this case, the circuit is arranged to detect either increases or decreases in the sensed radiation and in response thereto to provide a signal indicating the presence of an air bubble. The circuit could be arranged to store an indication of the type of liquid being supplied (which could for example be entered using the keyboard), and to detect only decreases or only increases in sensed radiation depending upon the liquid type.

If desired, the bubble detector 18 could be positioned to detect air bubbles passing through the part 50 of the conduit 12, rather than the tube 56.

The sensor 18 may alternatively comprise a pair of capacitor plates positioned on respective sides of the tube 56. If an air bubble flows along the tube, the capacitance of the detector 18 will alter as the bubble passes between the plates. This can be detected, and if the change in capacitance lasts sufficiently long an alarm signal can be generated.

It should be noted that the part 50 can be configured, e.g. with recesses, to permit the sensor arrangement 16 and/or the bubble detector 18 to be fitted thereto in a predetermined location, rather than relying on all the elements being located accurately with respect to the base 6 of the housing 4.

The arrangement described above is controlled by a control unit 14 shown in Figure 3. The control unit 14 comprises a microprocessor 66, e.g. type 80C48 supplied by Intel, having an input/output bus 68 coupled to a programmable timer or counter 70 operable to generate output signals at regular intervals. The bus 68 is also coupled, via appropriate interface circuitry 72, to a digital display 74, warning lights 76 and 78, a keyboard 80, the stepper motor 84 an audio transducer 86, the heat source 53, the sensors 62 and 64, and the bubble detector 18.

The apparatus may be arranged to operate as follows. After the apparatus has been turned on by a switch of the keyboard 80, or automatically by the closing of the lid 8 with a reservoir 10 in position, the microprocessor 66 begins to operate in an active mode. The microprocessor detects the operating of keys of the keyboard 80, so that the user can enter a digital value representative of desired flow rate, which is displayed on the display 74. The microprocessor causes the source 53 to be pulsed, and detects the flow rate using the signals from sensor 62 and 64. If this differs from that set by the keyboard 80, the microprocessor generates a signal to cause the motor 84 to shift the position of the regulating member 92 either to increase or decrease the flow rate by an amount depending upon the variation of the flow rate from the set rate. The microprocessor again detects the flow rate using the source 53 and sensors 62 and 64, and continues to operate the motor 84 until the flow rate is close to the set rate. Due to the fact that the time taken for a sensing operation may vary considerably with different flow rates, the time between successive sensing operations may, for example, be of the order of one minute.

The microprocessor 66 then sends a counter set value along bus 68 to the counter 70. The microprocessor then switches into a standby mode, during which it consumes a relatively small amount of current. The counter 70 continues to operate during this period. The set value is continuously decremented, and when it reaches zero a signal is output on a line 88 to cause the microprocessor to enter its active mode. At this point the source 53 is energised and the outputs from sensors 62 and 64 checked to determine the flow rate. If this has departed from the set value, the motor 84 is again energised to alter the flow regulation by an amount dependent on the detected variation. The count value is again delivered to the counter 70 and the standby mode entered. However, if the departure from the set rate exceeds a predetermined amount, the microprocessor instead remains in its active mode so that regulation is carried out more rapidly.

The circuit 72 includes components for regularly checking the output from the bubble detector 18. At regular intervals, e.g. for 10 $\mu$s each millisecond, the source 110 is activated and the output from the sensor 112 checked by a comparator. If the output level is low, an interrupt signal is produced to put the microprocessor into its active mode. The warning lamp 76 is then illuminated. An audible alarm is also sounded, using the audio transducer 86. This arrangement could be modified so that the signal for placing the microprocessor in its active mode, or the signals generated to actuate the warning lamp 76 and audio transducer 86, are produced only if the size of the air bubble exceeds a predetermined size.

Whenever the microprocessor enters its active mode it checks the flow rate. If this is substantially different from the set rate and cannot quickly be corrected by operation of the regulator, the warning light 78 is operated and the alarm sounded, because this may be an indication that the liquid leaving the infusion apparatus is not being properly delivered to the patient.

The above arrangement may be modified so that the circuit 72 continually checks the output of the sensor arrangement 16, this circuit causing the microprocessor to enter its active mode upon detecting that an alarm needs to be given. The circuit may instead operate periodically,in response to a timer. In this arrangement, the microprocessor becomes active if an alarm is to be given or if a signal is output from counter 70, but alternatively the microprocessor can be caused to enter its active state only when an alarm needs to be given or the flow rate needs to be adjusted. This would avoid the need for a counter 70 to cause the microprocessor to enter its active mode.

The microprocessor 66 monitors the total amount of liquid delivered to the patient. This can be achieved by digitally integrating the detected flow rate with time. The microprocessor 66 is preferably capable of causing the total amount of liquid delivered by the apparatus to be displayed on the display 74, and/or permitting a user to set a permitted volume using the keyboard 80, following delivery of which the microprocessor 66 automatically shuts-off further liquid flow. In addition, the microprocessor 66 preferably gives an indication when the detected volume approaches the total volume initially stored in the reservoir, so as to indicate that replacement of the reservoir is required.

Various modifications to the system are possible. For example, instead of using the heat source 53, a microwave energy source could be used, or the part 50 could incorporate a conductor in which eddy currents are generated to heat the liquid. The heat need not be applied to the liquid in the part 50. There may be occasions on which it is desired to inject simultaneously more than one type of liquid, and for this purpose the housing 4 may be provided with an inlet for receiving the output flow from a similar apparatus, the inlet directing this flow into the conduit 12. Alternatively, the housing 4 may be designed to support two reservoirs, and have a respective sensor arrangement 16, regulator 20 and, if desired, bubble detector 18 for each of the reservoirs. These could all be coupled to the same control unit 14. The conduits 56 would then be linked at a point downstream of the regulators.

Although the above preferred embodiment uses a mechanical spring for pressurising the reservoir, it will be appreciated that many of the advantages can still be achieved if the apparatus is modified so that liquid flow is produced by gravity.

**Claims**

1. Medical infusion apparatus comprising:
   a liquid reservoir (10);
   a conduit (12) for delivering liquid from the reservoir (10) to a patient; and
   a monitor (16,53) for monitoring the flow rate of said liquid along said conduit (12);
   characterised in that said monitor comprises means (53) external of said conduit (12) for applying a pulse of heat to liquid at a location in the conduit (12) and radiation sensing means (16) disposed externally of the conduit (12) for sensing a change in temperature of liquid at a position downstream of the location, so that the speed of travel of the heat pulse can be determined, whereby the monitor (16,53) is non-intrusive to permit removal of the conduit (12) from the monitor (16,53).

2. Apparatus as claimed in claim 1, wherein said radiation sensing means (16) is operable to sense temperature changes at two positions spaced along the length of the conduit (12), whereby the time taken for the heat pulse to pass from said first position to said second position can be determined.

3. Apparatus as claimed in claim 1 or 2, wherein said means (53) for applying a pulse of heat comprises a member of resistive material in proximity to the conduit (12).

4. Apparatus as claimed in any preceding claim wherein said means (53) for applying a pulse of heat is in resilient engagement with said conduit (12).

5. Apparatus as claimed in claim 4, wherein the means (53) for applying a pulse of heat comprises a resilient clip for retaining the conduit.

6. Apparatus as claimed in any preceding claim, wherein the sensing means (16) comprises at least one infra-red sensor (62,64).

7. Apparatus as claimed in any preceding claim, wherein the apparatus includes a support means (4) for supporting said conduit (12) and said flow monitor (16,53), said conduit (12) comprising a unitary, integrally-formed insertion member (50) having first and second portions, said first portion (52) being adapted for insertion into said reservoir (10), and said member (50) being removable from said support means (4), and being configured such that, when carried by said support means (4), said second portion of said member (50) is located in a predetermined position with respect to the flow monitor (16,53), whereby the flow monitor can monitor the rate of flow of liquid through said second portion.

8. Apparatus as claimed in claim 7, wherein said member (50) is made of material which is substantially transparent to infra-red radiation.

9. Apparatus as claimed in any preceding claim, further comprising resilient pressurising means (40) which can be manually caused to store energy which is then used for applying pressure to said reservoir (10) to cause liquid to flow from said reservoir (10) along said conduit (12).

10. Apparatus as claimed in claim 9, wherein the resilient pressurising means (10) comprises a spring.

11. Apparatus as claimed in claim 9 or 10, further comprising an enclosure (4) containing said reservoir (10), said enclosure (4) having an access member (8) which can be opened to permit access for removal of the reservoir (10), means (42,46) being provided for withdrawing the resilient pressurising means (40) from the reservoir (10) in response to opening of said access member (8), so as to facilitate the removal of the reservoir (10) from said enclosure (4).

12. Apparatus as claimed in claim 11, wherein said withdrawing means (42,46) is arranged to cause energy to be stored by said pressurising means during withdrawal thereof.

13. Apparatus as claimed in any preceding claim, including a regulator (20) for regulating the flow of liquid along the conduit (12) in response to the monitored flow rate.

14. Apparatus as claimed in claim 13, the regulator (20) having an adjustable position for regulating the flow of liquid from the reservoir (10), and a motor (84) which can be energised to adjust said regulator position, the regulator position being stable while the motor (84) is de-energised.

15. Apparatus as claimed in claim 13 or 14, including a microprocessor (66) which is responsive to the monitored flow rate in order to control the regulator (20), the microprocessor (66) having an active mode in which it performs processing operations, and a standby mode in which it consumes less current than in the active mode, the apparatus further including means (70,18) for causing the microprocessor to enter its active mode in response either to elapsing of a predetermined interval or to detection of a disturbance in the flow of liquid, so that the microprocessor (66) can then determine and if necessary regulate the flow rate.

16. Apparatus as claimed in claim 15, including means (18) for sensing disturbances in the flow of liquid caused by the appearance of air bubbles in order to cause the microprocessor to enter its active mode.

17. Apparatus as claimed in any one of claims 13 to 16 when dependent on any one of claims 9 to 12, said apparatus further comprising a housing (4), said housing (4) supporting said reservoir (10), said conduit (12), said monitor (16,53), said pressurising means (40) and said regulating means (20).

18. Apparatus as claimed in any preceding claim, said apparatus further comprising control means (14) for intermittently operating the monitor (16,53) at predetermined intervals.

19. Apparatus as claimed in claim 18, wherein said control means (14) has first and second operational modes, said predetermined intervals being shorter in said first operational mode than in said second operational mode.

**Patentansprüche**

1. Medizinische Infusionsvorrichtung mit folgenden Bauteilen;
Flüssigkeitsbehälter (10);
Rohrleitung (12) zum Zuführen von Flüssigkeit aus dem Behälter (10) zum Patienten und Überwachungseinrichtung (16,53) zum Überwachen der Durchflußmenge der Flüssigkeit durch die Rohrleitung (12);
**dadurch gekennzeichnet, daß** die Überwachungseinrichtung eine Einrichtung (53) außerhalb der Rohrleitung (12) zum Anlegen eines Wärmeimpulses an die Flüssigkeit an einer Stelle in der Rohrleitung (12) und eine Strahlungstasteinrichtung (16) enthält, die außerhalb der Rohrleitung angeordnet ist, um eine Temperaturänderung der Flüssigkeit an einer Position stromab der Stelle abzutasten, so daß die Ausbreitungsgeschwindigkeit des Wärmeimpulses bestimmt werden kann, wobei die Überwachungseinrichtung (16,53) nicht eindringt, um eine Entfernung der Rohrleitung (12) von der Überwachungseinrichtung (16,53) zu ermöglichen.

2. Vorrichtung nach Anspruch 1, in der die Strahlungstasteinrichtung (16) wirksam ist, um Temperaturänderungen an zwei Positionen abzutasten, die entlang der Rohrleitung (12) im Abstand angeordnet sind, wodurch die Zeit bestimmt werden kann, die der Wärmeimpuls braucht, um von der ersten Position zur zweiten zu gelangen.

3. Vorrichtung nach Anspruch 1 oder 2, in der die Einrichtung (53) zum Anlegen eines Wärmeimpulses ein Bauteil aus Widerstandsmaterial in der Nähe der Rohrleitung (12) aufweist.

4. Vorrichtung nach einem vorangegangenen Anspruch, in der die Einrichtung (53) zum Anlegen eines Wärmeimpulses mit der Rohrleitung (12) federnd nachgiebig in Arbeitsberührung steht.

5. Vorrichtung nach Anspruch 4, in der die Einrichtung (53) zum Anlegen eines Wärmeimpulses eine federnd nachgiebige Klemme zum Festhalten der Rohrleitung ist.

6. Vorrichtung nach einem vorangegangenen Anspruch, in der die Tastvorrichtung (16) wenigstens einen Infrarotsensor (62,64) enthält.

7. Vorrichtung nach einem vorangegangenen Anspruch, die eine Haltevorrichtung (4) hat, um die Rohrleitung (12) und die Durchflußüberwachungseinrichtung (16,53) aufzunehmen, wobei die Rohrleitung ein als Einheit aus einem Stück ausgebildetes Einsetzbauteil (50) aufweist, das erste und zweite Abschnitte aufweist, wobei der erste Abschnitt (52) in den Behälter (10) eingesetzt und das Einsetzbauteil (50) aus der Haltevorrichtung (4) entfernt werden kann und so beschaffen ist, daß der zweite Abschnitt des Einsetzbauteils (50), wenn er durch die Haltevorrichtung (4) getragen wird, sich in einer vorbestimmten Position gegenüber der Durchflußüberwachungseinrichtung (16,53) befindet, wodurch sie die Durchflußmenge der Flüssigkeit durch den zweiten Abschnitt überwachen kann.

8. Vorrichtung nach Anspruch 7, in der das Einsetzbauteil (50) aus Material hergestellt ist, das im wesentlichen für Infrarotstrahlung durchlässig ist.

9. Vorrichtung nach einem vorangegangenen Anspruch, die weiterhin ein federnd nachgiebiges Druckmittel (40) aufweist, das manuell betätigbar ist, um Energie zu speichern, die zur Druckbeaufschlagung des Behälters (10) verwendet wird, damit Flüssigkeit aus dem Behälter (10) durch die Rohrleitung (12) fließt.

10. Vorrichtung nach Anspruch 9, in der das federnd nachgiebige Druckmittel (40) eine Feder aufweist.

11. Vorrichtung nach Anspruch 9 oder 10 hat weiterhin ein Gehäuse (4), das den Behälter (10) aufnimmt, wobei das Gehäuse (4) ein Zugangsbauteil (8) aufweist, das geöffnet werden kann, um einen Zugang für das Entfernen des Behälters (10) zu ermöglichen, wobei Vorrichtungen (42,46) vorgesehen sind, die als Reaktion auf das Öffnen des Zugangsbauteils (8) das federnd nachgiebige Druckmittel (40) vom Behälter (10) zurückzieht und so das Entfernen des Behälters (10) aus dem Gehäuse (4) erleichtert.

12. Vorrichtung nach Anspruch 11, in der die Zurückziehvorrichtung (42,46) so angeordnet ist, daß während ihres Zurückziehens Energie durch das Druckmittel gespeichert wird.

13. Vorrichtung nach einem vorangegangenen Anspruch mit einer Reguliervorrichtung (20) zum Regulieren des Flüssigkeitsdurchflusses durch die Rohrleitung (12) im Ansprechen auf die überwachte Durchflußmenge.

14. Vorrichtung nach Anspruch 13, wobei die Reguliervorrichtung (20) eine einstellbare Stellung zum Regulieren des Flüssigkeitsdurchflusses aus dem Behälter (10) einnimmt und einen Motor (84) aufweist, der erregbar ist, um die Reguliervorrichtungsstellung einzustellen, wobei die Stellung der Reguliervorrichtung unverändert bleibt, während der Motor (84) entregt ist.

15. Vorrichtung nach Anspruch 13 oder 14, die einen Mikroprozessor (66) hat, der auf die überwachte Durchflußmenge anspricht, um die Reguliervorrichtung (20) zu steuern, wobei der Mikroprozessor (66) einen Arbeitsmodus, bei dem er Rechenoperationen durchführt und einen Bereitschaftsmodus hat, in dem er weniger Strom als im Arbeitsmodus verbraucht, wobei die Vorrichtung weiterhin Vorrichtungen (70,18) enthält, die den Mikroprozessor bei Verstreichen eines vorbestimmten Intervalls oder bei Auffindung einer Störung beim Durchfluß der Flüssigkeit veranlaßt, seinen Arbeitsmodus einzuschalten, so daß der Mikroprozessor (66) die Durchflußmenge bestimmen und, wenn nötig, regulieren kann.

16. Vorrichtung nach Anspruch 15, die eine Vorrichtung (18) zum Abtasten von durch das Auftreten von Luftblasen verursachten Störungen beim Durchfluß der Flüssigkeit enthält, um den Mikroprozessor zu veranlassen, in seinen Arbeitsmodus zu schalten.

17. Vorrichtung nach einem der Ansprüche 13 bis 16, wenn von einem der Ansprüche 9 bis 12 abhängig, wobei die Vorrichtung weiterhin ein Gehäuse (4) aufweist, das den Behälter (10), die Rohrleitung (12), die Überwachungseinrichtung (16,53), das Druckmittel (40) und die Reguliervorrichtung (20) aufnimmt.

18. Vorrichtung nach einem vorangegangenen Anspruch, die weiterhin ein Steuerungsmittel (14) aufweist, das die Überwachungseinrichtung (16,53) im Abstand von vorbestimmten Intervallen in Betrieb setzt.

19. Vorrichtung nach Anspruch 18, in der das Steuerungsmittel (14) erste und zweite Betriebsmodi aufweist, wobei die vorbestimmten Intervalle bei der ersten Betriebsart kürzer als bei der zweiten sind.

## Revendications

1. Appareil d'infusion médicale comprenant:
   un réservoir (10) à liquide;
   un conduit (12) pour transporter le liquide depuis le réservoir (10) jusqu'à un patient; et
   un dispositif de contrôle (16, 53) pour contrôler le débit dudit liquide le long dudit conduit (12);
   caractérisé en ce que ledit dispositif de contrôle comprend des moyens (53) extérieurs audit conduit (12) pour appliquer une impulsion de chaleur audit liquide en un emplacement situé dans le conduit (12), et des moyens (16) de détection de rayonnement disposés à l'extérieur du conduit (12) pour détecter un changement de la température du liquide au niveau d'une position située en aval de l'emplacement, de façon à ce que la vitesse de déplacement de l'impulsion de chaleur puisse être déterminée, le dispositif de contrôle (16, 53) étant du type non intrusif pour permettre de retirer le conduit (12) du dispositif de contrôle (16, 53).

2. Appareil selon la revendication 1, dans lequel lesdits moyens de détection de rayonnement (16) sont aptes à détecter des changements de température au niveau de deux positions situées à distance l'une de l'autre le long du conduit (12), si bien que le temps mis par l'impulsion de chaleur pour passer de ladite première position à ladite seconde position peut être déterminé.

3. Appareil selon la revendication 1 ou la revendication 2, dans lequel lesdits moyens (53) d'application d'une impulsion de chaleur comprennent un élément en matière résistive situé à proximité du conduit (12).

4. Appareil selon l'une quelconque des revendications précédentes, dans lequel lesdits moyens (53) d'application d'une impulsion de chaleur sont engagés de façon élastique avec ledit conduit (12).

5. Appareil selon la revendication 4, dans lequel les moyens (53) d'application d'une impulsion de chaleur comprennent un collier élastique servant à retenir le conduit.

6. Appareil selon l'une quelconque des revendications précédentes, dans lequel les moyens de détection (16) comprennent au moins un détecteur de rayonnement infrarouge (62, 64).

7. Appareil selon l'une quelconque des revendications précédentes, ledit appareil comprenant des moyens formant support (4) pour supporter ledit conduit (12) et ledit appareil de contrôle de débit (16, 53), ledit conduit (12) comprenant un élément d'insertion unitaire formé de façon solidaire (50), comportant une première et une seconde parties, ladite première partie (52) étant conçue en vue d'être insérée dans ledit réservoir (10) et ledit élément (50) pouvant être retiré desdits moyens formant support (4), et étant configuré de telle manière que, lorsqu'il est supporté par lesdits moyens formant support (4), ladite seconde partie dudit élément (50) est située dans une position prédéterminée par rapport au dispositif de contrôle de débit (16, 53), si bien que le dispositif de contrôle de débit peut contrôler le débit du liquide circulant à travers ladite seconde partie.

8. Appareil selon la revendication 7, dans lequel ledit élément (50) est en une matière qui laisse sensiblement passer le rayonnement infrarouge.

9. Appareil selon l'une quelconque des revendications précédentes, comprenant en outre des moyens élastiques de mise sous pression (40) auxquels il est possible de faire emmagasiner de l'énergie manuellement, cette énergie étant ensuite utilisée pour appliquer une pression audit réservoir (10) pour entraîner la circulation du fluide le long dudit conduit (12) depuis ledit réservoir (10).

10. Appareil selon la revendication 9, dans lequel les moyens élastiques de mise sous pression se composent d'un ressort.

11. Appareil selon la revendication 9 ou la revendication 10, comprenant en outre une enceinte (4) contenant ledit réservoir (10), ladite enceinte (4) comportant un élément (8) d'accès qui peut être ouvert pour offrir un accès en vue de retirer le réservoir (10), des moyens (42, 46) étant prévus pour retirer du réservoir lesdits moyens élastiques de mise sous pression (40) en réponse à l'ouverture dudit élément (8) d'accès, de façon à faciliter le retrait du réservoir (10) hors de ladite enceinte (4).

12. Appareil selon la revendication 11, dans lequel lesdits moyens de retrait (42, 46) sont conçus de façon à ce que lesdits moyens de mise sous pression emmagasinent de l'énergie pendant leur retrait.

13. Appareil selon l'une quelconque des revendications précédentes, comprenant un régulateur (20) pour régler le débit de liquide le long du conduit (12) en réponse au débit contrôlé.

14. Appareil selon la revendication 13, le régulateur (20) ayant une position réglable pour régler le débit de liquide en provenance du réservoir (10), et un moteur (84) qui peut être activé en vue d'ajuster ladite position du régulateur, la position du régulateur étant stable pendant que le moteur (84) est désactivé.

15. Appareil selon la revendication 13 ou la revendication 14, comprenant un microprocesseur (66) qui commande le régulateur (20) en réponse au débit contrôlé, le microprocesseur (66) ayant un mode actif dans lequel il effectue les opérations de traitement, et un mode d'attente dans lequel il consomme moins de courant que dans le mode actif, l'appareil comprenant en outre des moyens (70, 18) pour faire entrer le microprocesseur dans son mode actif en réponse soit à l'écoulement d'un intervalle de temps prédéterminé, soit à la détection d'une perturbation dans l'écoulement du liquide, de sorte que le microprocesseur (66) peut alors déterminer et, en cas de besoin, régler le débit.

16. Appareil selon la revendication 15, comprenant des moyens (18) pour détecter des perturbations dans le débit de liquide dues à l'apparition de bulles d'air afin de faire en sorte que le microprocesseur entre dans son mode actif.

17. Appareil selon l'une quelconque des revendications 13 à 16, lorsqu'elles dépendent de l'une quelconque des revendications 9 à 12, ledit appareil comprenant en outre un logement (4), ledit logement (4) supportant ledit réservoir (10), ledit conduit (12), ledit dispositif de contrôle (16, 53), lesdits moyens de mise sous pression (40) et lesdits moyens formant régulateur (20).

18. Appareil selon l'une quelconque des revendications précédentes, ledit appareil comprenant en outre des moyens de commande (14) pour faire fonctionner de façon intermittente le dispositif de contrôle (16, 53) à intervalles prédéterminés.

19. Appareil selon la revendication 18, dans lequel lesdits moyens de commande (14) comportent un premier et un second modes opérationnels, lesdits intervalles prédéterminés étant plus courts dans ledit premier mode opérationnel que dans ledit second mode opérationnel.

# FIG.1.

# FIG.2.

14

# FIG. 3.

84

90

94

20

FIG.4.

92

56

102

104

96

98

18

110

FIG.5.

56

112